# EUROPEAN PATENT APPLICATION

(11) **EP 1 430 866 A1**
(43) Date of publication of application: **23.06.2004**
(21) Application number: 03028212.3
(22) Date of filing: 09.12.2003
(51) Int. Cl.: A61K 6/083, C08K 5/00, C08F 230/02

(54) **Coating material composition for dental prosthesis**

(30) Priority: 18.12.2002 JP 2002366488
(71) Applicant: GC Corporation, Itabashi-ku, Tokyo 174 (JP)
(72) Inventor: Nakabayashi, Nobuo, Matsudo-shi Chiba-ken (JP); Miyano, Tatsunosuke, Itabashi-ku Tokyo (JP); Kumagai, Tomohiro, Itabashi-ku Tokyo (JP)
(74) Representative: Müller-Boré & Partner Patentanwälte

(57) **Abstract**

In order to suppress adhesion of oral contamination substances such as protein and oral bacteria causing oral disorders such as ozostomia on a surface of a dental prosthesis, so as to prevent a biofilm such as dental plaque from being formed thereon, the coating material composition for a dental prosthesis comprises 1 to 150 g/l of a copolymer of 2-methacryloyloxyethylphosphorylcholine and a (meth)acrylate contained in at least one solvent selected from water, ethanol, propanol, butanol, pentanol and acetone, an amount of the 2-methacryloyloxyethylphosphorylcholinecomponentin the copolymer being 3 to 90% by mole.

## Description

The present invention relates to a coating material composition for a dental prosthesis capable of forming, on a surface of a dental prosthesis, such a coating layer suppressing protein and oral bacteria from being adhered.

A dental prosthesis, such as a denture, a bridge and a crown prosthesis, is used for remedying deterioration or loss of oral functions and defect in shape. The dental prosthesis is used in an oral cavity for a long period of time, and therefore, a biofilm, such as dental plaque, is formed thereon due to adhesion of oral contamination substances (formed mainly with protein in saliva, and bacteria, e.g., Streptococcus mutans and fungi). The biofilm causes oral disorders, such as ozostomia, inflammation of oral mucosa and stomatitis, and therefore, it is desirably removed as early as possible after the formation thereof.

What are widely practiced as a method for removing a biofilm on a dental prosthesis include brushing with a toothbrush or the like, and in the case of a plate denture, include such a method in that a dental prosthesis is immersed in chemicals, such as a denture detergent, and the biofilm is removed by breaking it by utilizing oxygen and an enzyme contained in the chemicals. These methods are for removing a biofilm having been formed on a dental prosthesis to remove proteins and oral bacteria adhered thereto, as early as possible. However, once forming a biofilm, it is difficult to remove it from a dental prosthesis by the removing method with brushing or chemicals. Therefore, it has been difficult to attain a considerable effect on prevention of oral disorders because a biofilm is liable to remain even after brushing or immersion in chemicals.

As a method for suppressing the formation of a biofilm, such a method is proposed in that an antibacterial agent is added to a material of a dental prosthesis. However, an antibacterial agent exerts only an effect of suppressing growth of bacteria, such as oral bacteria and fungi, adhered to the dental prosthesis, and therefore, a biofilm cannot be prevented from being formed.

Under the circumstances, antibacterial preparations exerting excellent antibacterial activity against an aggregation or an agglomerate of bacteria, such as a biofilm and plaque, to which a single antibacterial agent is difficult to act, are proposed, i.e., an antibacterial preparation containing arginine or a derivative thereof, a compound exerting antibacterial activity and a surface active agent (as described, for example, in JP-A-8-151324), an antibacterial preparation containing lysine or a derivative thereof, a compound exerting antibacterial activity and a surface active agent (as described, for example, in JP-A-8-151325), and an antibacterial preparation containing histidine or a derivative thereof, a compound exerting antibacterial activity and a surface active agent (as described, for example, in JP-A-8-151326). However, these antibacterial preparations exert excellent antibacterial activity against an aggregation or an agglomerate of bacteria, such as a biofilm and plaque, having been present in an oral cavity, but are not intended to prevent a biofilm from being formed.

An object of the present invention is to provide such a coating material composition for a dental prosthesis that is to suppress adhesion of oral contamination substances, such as protein and oral bacteria, causing oral disorders, such as ozostomia, on a surface of a dental prosthesis, so as to prevent a biofilm from being formed thereon.

As a result of earnest investigations made by the inventors for solving the problems associated with the conventional art, the inventors have noted that the surface of the biological membrane constituting the biological tissue is considerably excellent in non-absorbability and non-activation property of protein and oral bacteria, and it has been found that in the case where a copolymer obtained from 2-methacryloyloxyethylphosphorylcholine and a hydrophobic (meth)acrylate is particularly used as a coating material component among the components having the same structures as the polar group of phospholipid, which is the major component of the biological membrane, a coating layer having the non-absorbability and the non-activation property, which is safe for living bodies, canbe formed on a surface of a dental prosthesis, so as to suppress adhesion of protein and oral bacteria. Thus, the present invention has been completed.

The present invention relates to a coating material composition for a dental prosthesis comprising 1 to 150 g/l of a copolymer of 2-methacryloyloxyethylphosphorylcholine and a (meth)acrylate contained in at least one solvent selected from water, ethanol, propanol, butanol, pentanol and acetone, an amount of the 2-methacryloyloxyethylphosphorylcholinecomponentin the copolymer being 3 to 90% by mole.

The copolymer used in the present invention is a copolymer of 2-methacryloyloxyethylphosphorylcholine and a (meth)acrylate having a structure represented by the following chemical formula: 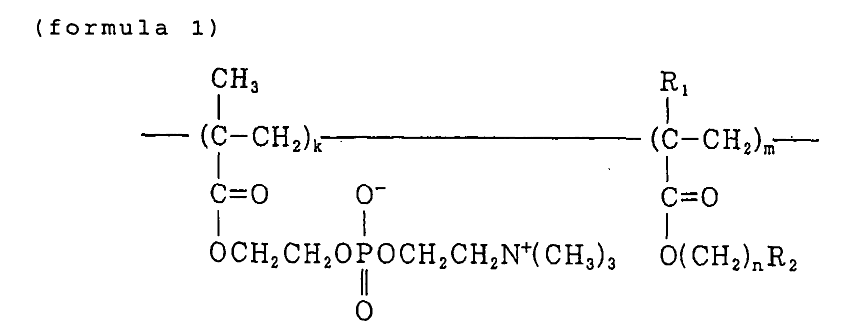 wherein k represents a number of 0.03 to 0.90; m represents a number of 0.10 to 0.97; n represents an integer of 1 or more; R₁ represents a hydrogen atom or a methyl group; and R₂ represents a hydrogen atom or an OR' group (wherein R' represents an aliphatic hydrocarbon group or an aromatic hydrocarbon group).

Examples of the aliphatic hydrocarbon group represented by R' of the (meth)acrylate as a copolymerization component include an alkyl group and an alkenyl group, and examples of the aromatic hydrocarbon group represented by R' include a phenyl group.

Specific examples of the (meth) acrylate include methyl methacrylate, propyl methacrylate, butyl methacrylate, pentyl methacrylate, hexyl methacrylate, heptyl methacrylate, octyl methacrylate, tridecyl methacrylate, stearyl methacrylate, 2-ethoxyethyl methacrylate, 2-ethoxypropyl methacrylate, 2-phenoxyethyl methacrylate, 2-butoxyethyl methacrylate, and acrylates obtained by substituting the methacrylic acid residual groups thereof with acrylic acid residual groups.

The molecular weight of the copolymer of 2-methacryloyloxyethylphosphorylcholine and a (meth)acrylate may vary depending on the conditions where the copolymer is used, and the molecular weight is preferably 5,000 to 500,000, and more preferably 20,000 to 200, 000, from the standpoint of the strength of the resulting coating layer and the handleability of the composition.

The copolymer of 2-methacryloyloxyethylphosphorylcholine and a (meth)acrylate contained in the coating material composition for a dental prosthesis according to the present invention includes the 2-methacryloyloxyethylphosphorylcholine component in an amount of 3 to 90% by mole, and preferably 10 to 60% by mole. In the case where the amount of the 2-methacryloyloxyethylphosphorylcholinecomponentis less than 3% by mole, the effect of non-absorbability and non-activation property is difficultly obtained, and in the case where it exceeds 90% by mole, only a weak mutual interaction between the copolymer and the surface of the dental prosthesis occurs to fail to form a stable coating layer.

In the case where the copolymer used in the coating material composition for a dental prosthesis includes the 2-methacryloyloxyethylphosphorylcholine in a small amount of 3 to 40% by mole, there is such a tendency that the copolymer is hardly soluble in water, and it is preferred that the copolymer is used after being dissolved in ethanol or acetone.

The coating material composition for a dental prosthesis according to the present invention contains the copolymer of 2-methacryloyloxyethylphosphorylcholine and a (meth)acrylate in a concentration of 1 to 150 g/l, and more preferably 10 to 100 g/l, with respect to the solvent described later. In the case where the concentration of the copolymer of 2-methacryloyloxyethylphosphorylcholine and a (meth) acrylate is less than 1 g/l, such a coating layer cannot be obtained that is sufficient to provide the non-absorbability, and in the case where it exceeds 150 g/l, no further improvement of the effect can be obtained.

The solvent used in the coating material composition for a dental prosthesis according to the present invention is necessarily at least one selected from water, ethanol, propanol, butanol, pentanol and acetone, which are safe in an oral cavity. However, the concentration of the solvents other than water is preferably about 60% or less with the balance being water in the case where the composition is applied to such a dental prosthesis as a resin plate that may be discolored or cracked upon immersing in the solvents other than water for a long time, and a plate containing amucosal conditioner that may be swelled upon immersing for a long time.

The coating material composition for a dental prosthesis according to the present invention is used in such a manner that a dental prosthesis to be coated is immersed in the coating material composition for a dental prosthesis for 1 minute to overnight, and after being lightly washed with water depending on necessity, it is dried, or in alternative, the coating material composition is uniformly coated directly on the surface of the dental prosthesis with a brush or the like, and then the surface is dried.

The invention will be described in more detail with reference to the following examples, but the invention is not construed as being limited to the example.

### Copolymer of 2-Methacryloyloxyethylphosphorylcholine and (Meth)acrylate

Copolymers 1, 2 and 3 of 2-methacryloyloxyethylphosphorylcholine (MPC) and a methacrylate having the compositions shown in Table 1 below were prepared by using butyl methacrylate (BMA) , stearylmethacrylate (SMA) and ethylhexyl methacrylate (EHMA) as the (meth)acrylate.

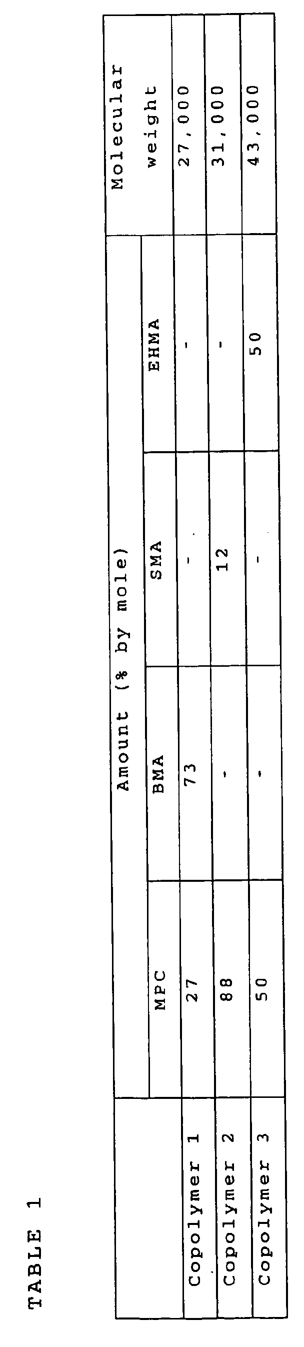

### Examples 1 to 6 and Comparative Examples 1 and 2

Coating material compositions used in Examples 1 to 6 were prepared according to the formulations shown in Table 2 below. A dental prosthesis used in Comparative Example 1 was a resin-made denture base plate having an antibacterial agent contained therein, and a dental prosthesis used in Comparative Example 2 was that having no coating material composition for a dental prosthesis on the surface thereof.

### Test for Adhesion of Oral Bacteria

As a denture base plate to be coated, a denture base plate was produced by a wet heating polymerization process, which had been ordinarily practiced, by using a material for a denture base plate (Acron, a trade name, produced by GC Corporation).

In Examples 1 and 3, the coating material compositions for a dental prosthesis shown in Table 2 were coated on the surface of the denture base plates with a thin paint brush and naturally dried at room temperature. In Examples 2 and 4, the denture base plates were immersed in the coating material compositions for a dental prosthesis shown in Table 2 for 60 minutes and naturally dried. In Examples 5 and 6, the denture base plates were immersed in the coating material compositions for a dental prosthesis shown in Table 2 for 5 minutes and naturally dried.

A denture base plate used in Comparative Example 1 was prepared in the same manner as in Example 1 except that the monomer liquid of the material for the denture base plate contains a cationic surface active agent (DC5700, a trade name, produced by Dow Corning, Inc.) as an antibacterial agent in an amount of 4%.

Evaluation was carried out for Streptococcus mutans, which directly affected formation of a biofilm in an oral cavity, by using a commercially available measurement kit for Streptococcus mutans (Dentocult SM, a trade name, produced by Orion Diagonostica, supplied by Eiko Corp.) in the following manner.

The denture base plates of Examples and Comparative Examples were placed in oral cavities of five subjects for 1 minute and then taken out from the oral cavities. Only Streptococcus mutans were cultivated for 48 hours byusing a culture medium annexed to the measurement kit, and the number of colonies in an area of 1 cm² on a central part of the respective denture base plates was counted with the naked eye to evaluate the state of adhesion of Streptococcus mutans. The number of colonies was evaluated with an average value of the five denture base plates. The results obtained are shown in Table 2.

### Test for fixing in Oral Cavity

Denture base plates produced with coating in the same manner as in Examples 1 to 6 were used as test specimens. Those for comparative examples were the same as in Comparative Examples 1 and 2.

The dentures were fixed on subjects for 8 hours, and then after being lightly washed with water, the weight increments of the denture base plates after fixing were measured. The measurement of weight was carried out for the respective denture base plates in a completely dry state before and after fixing, and average values were obtained by fixing the denture base plates of Examples and Comparative Examples in five subjects, respectively. The results obtained are shown in Table 2.

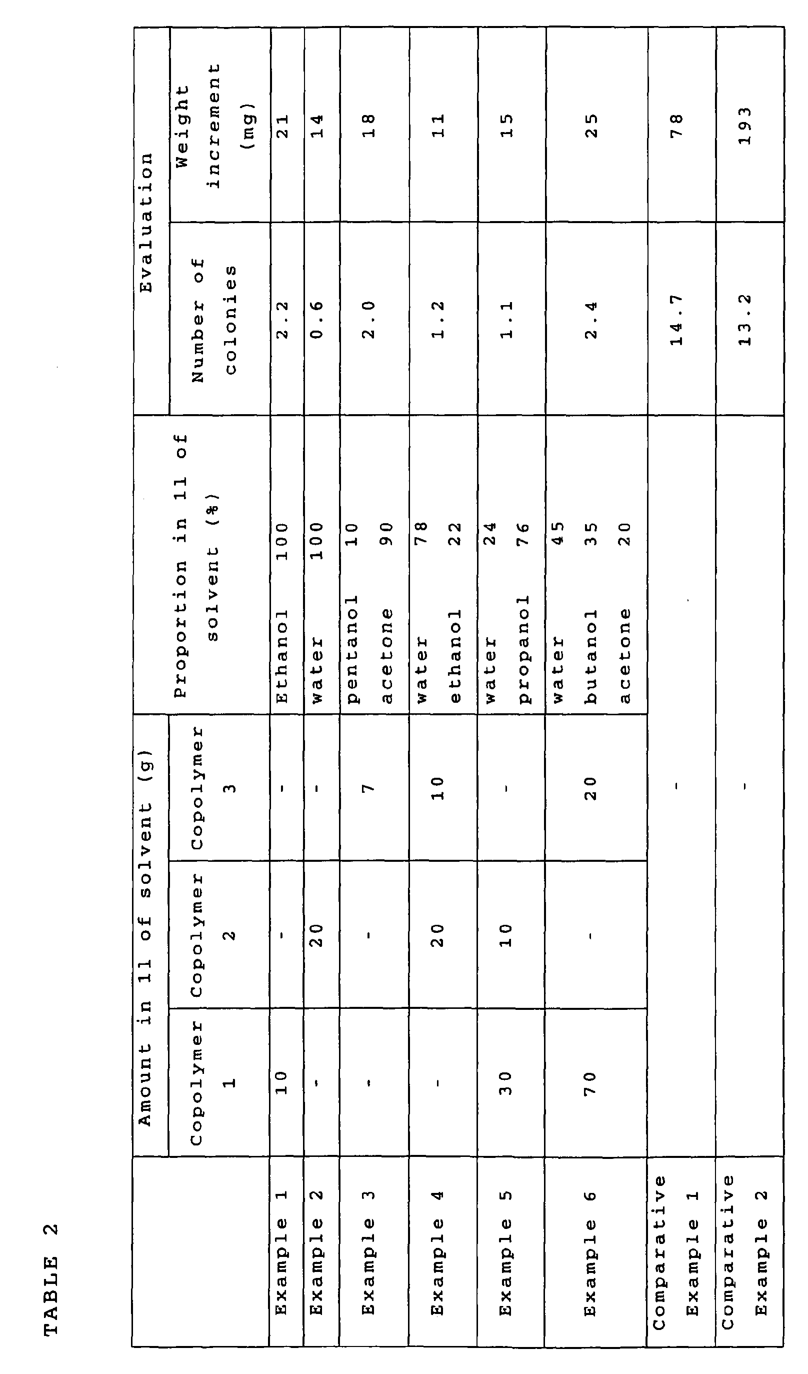

It was confirmed from the numbers of colonies shown in Table 2 that the coating material composition for a dental prosthesis according to the present invention had such an effect that adhesion of Streptococcus mutans due to contact of saliva was suppressed in comparison to the case where no coating was formed. It was also confirmed that, particularly, in the cases where the copolymer 2 containing a larger amount of 2-methacryloyloxyethylphosphorylcholine was used, the number of colonies was smaller, and thus a coating layer excellent in suppression of adhesion of bacteria was formed.

It was found that less weight increment after fixing occurred in the denture base plate having the coating material composition for a dental prosthesis according to the present invention applied thereto. It is considered that the weight increment of the denture base plate includes the amount of contamination substances attached during the period of having been fixed in the oral cavity, and therefore, it is understood that less adhesion of contamination substances occurs in Example. On the other hand, the weight increment of Comparative Example 1 where the antibacterial agent was added was smaller than Comparative Example 2 having no coating but was obviously larger than those of Examples. It is thus understood that an antibacterial agent may promise an effect on suppression of growth of bacteria attached to a denture base plate but provides less effect on suppression of adhesion of contamination substances, such as bacteria and protein.

As described in detail in the foregoing, the coating material composition for a dental prosthesis according to the present invention suppresses adhesion of oral contamination substances, such as protein and oral bacteria, causing oral disorders, such as ozostomia, on a surface of a dental prosthesis, so as to prevent a biofilm from being formed thereon.

Accordingly, thecoatingmaterialcompositionfor a dental prosthesis according to the present invention provides significant values by contributing to the field of dentistry.

## Claims

1. A coating material composition for a dental prosthesis comprising 1 to 150 g/l of a copolymer of 2-methacryloyloxyethylphosphorylcholine and a (meth)acrylate contained in at least one solvent selected from water, ethanol, propanol, butanol, pentanol and acetone, having an amount of the 2-methacryloyloxyethylphosphorylcholinecomponentin the copolymer being 3 to 90% by mole.
